# EUROPEAN PATENT SPECIFICATION

(11) **EP 1 345 584 B1**
(45) Date of publication and mention of the grant of the patent: **22.07.2009**
(21) Application number: 01991271.6
(22) Date of filing: 19.12.2001
(51) Int. Cl.: A61Q 15/00, A61K 8/58, A61K 8/81, A61K 8/92

(54) **HIGH EFFICACY ANTIPERSPIRANT STICK**
HOCHWIRKSAMER ANTITRANSPIRANT-STIFT
BATONNET ANTISUDORIFIQUE À HAUTE EFFICACITÉ

(30) Priority: 21.12.2000 US 257270 P; 09.11.2001 US 35383 P
(43) Date of publication of application: 24.09.2003
(73) Proprietor: Colgate-Palmolive Company, New York NY 10022-7499 (US)
(72) Inventor: GUENIN, Eric, P., Pennington, NJ 08534 (US); MATTAI, Jairajh, Piscataway, NJ 08854 (US); GALE, Anne, Landing, NJ 07850 (US); HALL-PUZIO, Patricia, Succasunna, NJ 07876 (US); LEE, Wilson, Bloomfield, NJ 07003 (US)
(74) Representative: Jenkins, Peter David
(86) International application number: PCT/US2001/049009
(87) International publication number: WO 2002/049592

(56) References cited:
- WO-A-00/61081
- US-A- 5 885 559
- US-A- 5 989 531
- US-A- 6 051 216

## Description

### Field of the Invention

This invention relates to antiperspirant/deodorant stick products made without stearyl alcohol and which have higher efficacy and better aesthetics than stearyl alcohol gelled sticks. This application is related to provisional patent applications U.S. Serial Numbers 60/257266 and 60/257269 (Attorney Docket Numbers IR 6602 and 6603, respectively) which were filed on the same date as the parent case for this application U.S. Serial Number 60/257270 which was filed on December 21, 2000 as a provisional case. This case is also related to two U.S. Patent applications serial numbers not yet obtained (Attorney Reference Numbers IR 6602-00U and IR 6603-00U) being filed on the same day as this current patent application.

### Background of the Invention

There is a continuing trend to develop new and superior cosmetic compositions especially for the reduction and/or elimination of wetness and/or odor under the arms. Particular efforts include developing lower residue products especially with improved efficacy and aesthetics. Various product forms have included sticks (especially gel/sticks), gels, soft solids, roll-ons, aerosols and creams. Of these various forms the sticks, gels, soft solids creams and roll-ons are made with a liquid base material incorporating a solidifying agent and/or gelling agent and/or thickening agent. Generally, these forms include a solution of the cosmetically active ingredient in a suitable vehicle, a suspension of the active ingredient in a carrier vehicle, or a multiphasic dispersion or emulsion in which a solution of the active ingredient is dispersed or suspended in some continuous phase or in which the solubilized active ingredient constitutes the continuous phase.

One of the most frequently used gelling agents for stick products is stearyl alcohol. While it gives a solid product, it can reduce efficacy of the antiperspirant salt included in the formulation. This invention is a stick made without stearyl alcohol and which has an efficacy that is at least 10% better in sweat reduction than a stick that is gelled with stearyl alcohol.

Thus, it is an object of the invention to provide improved cosmetic compositions with the improvements as previously described and which are useful as antiperspirants and/or deodorants. These and other objects of the invention will be apparent from the following description of the invention.

### Summary of the Invention

It has been found that a high efficacy antiperspirant/deodorant stick product may be made by combining the following ingredients where all amounts are in weight percent based on the total weight of the composition:
(a) 30-70% volatile cyclomethicone (particularly 40-50%) (for example, D4, D5, D6 and mixtures of two or more of the foregoing);
(b) 10-25% of an antiperspirant active;
(c) 1-15% of an emollient (which may also be a mixture of two or more emollients) and which may include a non-volatile silicone (especially wherein the emollient is selected from the group consisting of C12-15 alkyl benzoate; and medium volatility dimethicone (especially 10-350 centistoke material and more especially 10-50 centistolce material);
(d) 1-14% of polyethylene (particularly 3-10%) comprising one or more members selected from the group consisting of homopolymers and copolymers of polyethylene wherein the polyethylene (i) is at least 90% linear; (ii) has a molecular weight in the range of 300-3000 (especially 300-1000 and more especially 300-500); (iii) has a melting point in the range of 50-129 degrees C (for example, 50-70 degrees C, 60-70 degrees C, and 70-129 degrees C); and (iv) has a polymer backbone of CH₃CH₂-(CH₂-CH₂)ₙ-CH₂-CH₃ (which can also be represented as CH₃CH₂-(CH₂-CH₂)ₙ-H), where n is an average number and is selected to be in the range of 10-106 (for example, polyethylenes sold under the PERFORMALENE name from New Phase Technology, Piscataway, NJ); and
(e) 0.3-7% of a wax (including a single was or a mixture of waxes) as a cogellant with the polyethylene wherein the wax has a melting point in the range of 40-97 degrees C (for example, 40-80 degrees C), and particularly wherein the wax is a member selected from the group consisting of Japan wax substitute 525 (from Ross Wax, Jersey City, NJ), Beeswax 136 (for example, from Ross Wax); and microcrystalline wax having a melting point in the range of 60-97 degrees C.;
provided that the ratio of wax:polyethylene is in the range of 1:1-1:10, particularly 1:2-1:10, and more particularly in a ratio of 3:8.

Other optional ingredients include 0.1-5% fragrance and an effective amount of an antimicrobial (for example, an antibacterial) agent.

### Detailed Description of the Invention

Various types of cyclomethicones may be used. Illustratively, and not by way of limitation, the volatile silicones are one or more members selected from the group consisting of cyclic polydimethylsiloxanes such as those represented by Formula I: 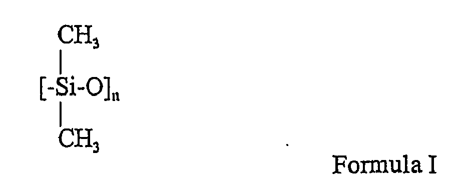 where n is an integer with a value of 3-7, particularly 5-6. By volatile silicone material is meant a material that has a measurable vapor pressure at ambient temperature. For example, DC-245 fluid or DC-345 fluid from Dow Corning Corporation (Midland, Michigan) is a type of cyclomethicone which can be used. These include a tetramer (or octylmethylcyclotetrasiloxane) and a pentamer (or decamethylcyclopentasiloxane).

The antiperspirant active can be selected from the group consisting of any of the known antiperspirant active materials. These include, by way of example (and not of a limiting nature), aluminum chlorohydrate, aluminum chloride, aluminum sesquichlorohydrate, zirconyl hydroxychloride, aluminum-zirconium glycine complex (for example, aluminum zirconium trichlorohydrex gly, aluminum zirconium pentachlorohydrex gly, aluminum zirconium tetrachlorohydrex gly and aluminum zirconium octochlorohydrex gly), aluminum chlorohydrex PG, aluminum chlorohydrex PEG, aluminum dichlorohydrex PG, and aluminum dichlorohydrex PEG. The aluminum-containing materials can be commonly referred to as antiperspirant active aluminum salts. Generally, the foregoing metal antiperspirant active materials are antiperspirant active metal salts. In the embodiments which are antiperspirant compositions according to the present invention, such compositions need not include aluminum-containing metal salts, and can include other antiperspirant active materials, including other antiperspirant active metal salts. Generally, Category I active antiperspirant ingredients listed in the Food and Drug Administration's Monograph on antiperspirant drugs for over-the-counter human use can be used. In addition, any new drug, not listed in the Monograph, such as aluminum nitratohydrate and its combination with zirconyl hydroxychlorides and nitrides, or aluminum-stannous chlorohydrates, can be incorporated as an antiperspirant active ingredient in antiperspirant compositions according to the present invention.

Particular types of antiperspirant actives include aluminum zirconium trichlorohydrex and aluminum zirconium tetrachlorohydrex either with or without glycine. A particular antiperspirant active is aluminum trichlorohydrex gly such as AZZ-902 SUF (from Reheis Inc., Berkley Heights, NJ) which has 98% of the particles less than 10 microns in size.

Antiperspirant actives can be incorporated into compositions according to the present invention in amounts in the range of 10-25% (on an actives basis) of the final composition, but the amount used will depend on the formulation of the composition. At lower levels the antiperspirant active material will not substantially reduce the flow of perspiration as effectively, but will reduce malodor, for example, by acting also as an antimicrobial material.

The antiperspirant active material is desirably included as particulate matter suspended in the composition of the present invention in amounts as described above, but can also be added as solutions or added directly to the mixture.

Emollients are a know class of materials in this art, imparting a soothing effect to the skin. These are ingredients which help to maintain the soft, smooth, and pliable appearance of the skin. Emollients are also known to reduce whitening on the skin and/or improve aesthetics. Examples of chemical classes from which suitable emollients can be found include:
(a) fats and oils which are the glyceryl esters of fatty acids, or triglycerides, normally found in animal and plant tissues, including those which have been hydrogenated to reduce or eliminate unsaturation. Also included are synthetically prepared esters of glycerin and fatty acids. Isolated and purified fatty acids can be esterified with glycerin to yield mono-, di-, and triglycerides. These are relatively pure fats which differ only slightly from the fats and oils found in nature. The general structure may be represented by Formula III: 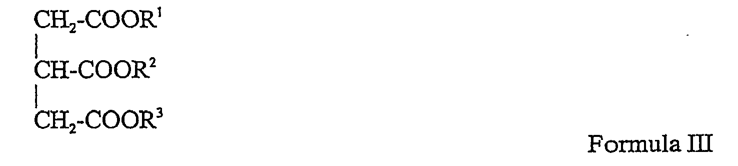 wherein each of R¹, R², and R³ may be the same or different and have a carbon chain length (saturated or unsaturated) of 7 to 30. Specific examples include peanut oil, sesame oil, avocado oil, coconut, cocoa butter, almond oil, safflower oil, corn oil, cotton seed oil, castor oil, hydrogenated castor oil, olive oil, jojoba oil, cod liver oil, palm oil, soybean oil, wheat germ oil, linseed oil, and sunflower seed oil;
(b) hydrocarbons which are a group of compounds containing only carbon and hydrogen. These are derived from petrochemicals. Their structures can vary widely and include aliphatic, alicyclic and aromatic compounds. Specific examples include paraffin, petrolatum, hydrogenated polyisobutene, and mineral oil.
(c) esters which chemically are the covalent compounds formed between acids and alcohols. Esters can be formed from almost all acids (carboxylic and inorganic) and any alcohol. Esters here are derived from carboxylic acids and an alcohol. The general structure would be R⁴CO-OR⁵. The chain length for R⁴ and R⁵ can vary from 7 to 30 and can be saturated or unsaturated, straight chained or branched. Specific examples include isopropyl myristate, isopropyl palmitate, isopropyl stearate, isopropyl isostearate, butyl stearate, octyl stearate, hexyl laurate, cetyl stearate, diisopropyl adipate, isodecyl oleate, diisopropyl sebacate, isostearyl lactase, C₁₂₋₁₅ alkyl benzoates, myreth-3 myristate, dioctyl malate, neopentyl glycol diheptanoate, neopentyl glycol dioctanoate, dipropylene glycol dibenzoate, C₁₂₋₁₅ alcohols lactate, isohexyl decanoate, isohexyl caprate, diethylene glycol dioctanoate, octyl isononanoate, isodecyl octanoate, diethylene glycol diisononanoate, isononyl isononanoate, isostearyl isostearate, behenyl behenate, C₁₂₋₁₅ alkyl fumarate, laureth-2 benzoate, propylene glycol isoceteth-3 acetate, propylene glycol ceteth-3 acetate, octyldodecyl myristate, cetyl ricinoleate, myristyl myristate.
(d) saturated and unsaturated fatty acids which are the carboxylic acids obtained by hydrolysis of animal or vegetable fats and oils. These have general structure R⁶COOH with the R⁶ group having a carbon chain length between 7-10 straight chain.
(e) lanolin and its derivatives which are a complex esterified mixture of high molecular weight esters of (hydroxylated) fatty acids with aliphatic and alicyclic alcohols and sterols. General structures would include R⁸CH₂-(OCH₂CH₂)ₙOH where R⁸ represents the fatty groups derived from lanolin and n=5 to 75 or R⁹CO-, (OCH₂CH₂)ₙOH where R⁹CO- represents the fatty acids derived from lanolin and n=5 to 100. Specific examples include lanolin, lanolin oil, lanolin wax, lanolin alcohols, lanolin fatty acids, isopropyl lanolate, ethoxylated lanolin and acetylated lanolin alcohols.
(f) alkoxylated alcohols wherein the alcohol portion is selected from aliphatic alcohols having 2-18 and more particularly 4-18 carbons, and the alkylene oxide portion is selected from the group consisting of ethylene oxide, and propylene oxide having a number of alkylene oxide units from 2-53 and, more particularly, from 2-15. Specific examples include PPG-14 butyl ether and PPG-53 butyl ether.
(g) silicones as the linear organo-substituted polysiloxanes which are polymers of silicon/oxygen with general structure:
   (1) (R¹⁰)₃SiO(Si (R¹¹)₂O)ₓSi(R¹²)₃ where R¹⁰, R¹¹ and R¹² can be the same or different and are each independently selected from the group consisting of phenyl and C1-C60 alkyl; or
   (2) HO(R¹⁴)₂SiO(Si (R¹⁵)₂O)ₓSi(R¹⁶)₂OH, where R¹⁴, R¹⁵ and R¹⁶ can be the same or different and are each independently selected from the group consisting of phenyl and C1-C60 alkyl;
   (with specific examples including dimethicone, dimethiconol behenate, C₃₀₋₄₅ alkyl methicone, stearoxytrimethylsilane, phenyl trimethicone and stearyl dimethicone); and
(h) mixtures and blends of two or more of the foregoing. One particular group of emollients includes C12-15 alkyl benzoate (FINSOLV TN from Finetex Inc., Elmwood Park, NJ), medium volatility dimethicone (especially 10-350 centistoke material and more especially 10-50 centistoke material), isopropyl myristate; and neopentyl glycol diheptanoate.

The emollient or emollient mixture or blend thereof incorporated in compositions according to the present invention can, illustratively, be included in amounts of 1-15%, and particularly 3 - 12 % by weight of the total weight of the composition.

Polyethylenes may be made in a variety of ways. Each polymerization method has its own advantages and disadvantages and may be used to obtain a polymer with specific properties. For example, free radical polymerization of ethylene using radical initiators usually gives highly branched polymers known as low-density polyethylene. This method usually requires high temperatures and pressures. Preparation of linear polyethylene can be achieved at low temperatures and pressures using transition metal compounds and organometallic compounds as a catalyst. Ziegler-Natta catalyst (for example, TiCl₄ and Al(C₂H₅)₃) is a widely used catalyst system for commercial preparation of linear polyethylene. The molecular weight of the polymer can be manipulated by controlling temperature, pressure and the ratios of the two-part catalyst system used. The molecular weight can also be controlled by using chain transfer agents such as molecular hydrogen and Zn(C₂H₅)₂. Active hydrogen compounds (for example, methanol) can also bring about termination of the growing chains just as they do in anionic polymerization.

The method for making both low and high molecular weight linear polyethylene is the same. Low molecular weight polymer is obtained by controlling the molecular weight using chain transfer agents such a hydrogen gas or methanol followed by isolation of the desired molecular weight through fractionation by distillation or reprecipitation with solvents of varying polarities. One can also use a catalyst system which employs a combination of transition metal compound or an element from Groups IV to VIII such as vanadium, chromium, or cobalt as well as an organometallic compound of a metal from Groups I and III of the periodic table. One typical example for making linear polyethylene is described below (see Example PE).

The polyethylenes useful in this invention include those sold under the PERFORMALENE^{™} product line (New Phase Technology, Piscataway, NJ); MARCUS polyethylenes (for example M200, M300, M500 and M4040) (Marcus Oil and Chemical, Houston, TX); HPWax polyethylene waxes (for example, HP CWP 200, HP CWP 500 and HP 400M) (Hase Petroleum Wax Co., Arlington Heights, IL). Mixtures of neutral polyethylene wax/polypropylene wax may also be used such as Polarwachs® PT30, Polarwachs® PT70, and Polycerit® AT-grades (TH. C. TROMM GmbH, Germany). Suitable polyethylenes may also be made using information found in the art such as British Patent 1 450 285.

The stick antiperspirant/deodorant products of this invention is an opaque product which leaves little or no white residue when applied and which exhibits improved efficacy and stability as compared to other stick formulations made with stearyl alcohol. Reduction of sweat of at least 10% more than that achieved with sticks gelled with stearyl alcohol can be achieved with the compositions of the invention.

Suitable antimicrobial agents include, for example, bacteriostatic quaternary ammonium compounds such as 2-amino-2-methyl-1-propanol (AMP), cetyltrimethylammonium bromide, cetyl pyridinium chloride, 2, 4, 4'-trichloro-2'-hydroxydiphenylether (Trielosan), N-(4-chlorophenyl)-N'-(3,4-dichlorophenyl)urea (Triclocarban), silver halides, octoxyglycerin (Sensiva^{™} SC 50) and various zinc salts (for example, zinc ricinoleate). The bacteriostat can, illustratively, be included in the composition in an amount of 0-5%, particularly 0.01-1.0% by weight, of the total weight of the composition. Triclosan can illustratively be included in an amount of from 0.05% to about 0.5% by weight, of the total weight of the composition.

A variety of fragrances can be used in these compositions if scented products are desired. Fragrances can be used in an amount in the range of 0-5%, particularly 0.01-2.0%, and, for example, at a level of 1%.

Masking agents can be used in an amount of 0.05-5.0% (particularly 0.05-2%) by weight based on the total weight of the composition if an unscented product is desired.

For additional hardening of sticks, other additives having a melting point in the range of 78-98 degrees C such as long chain alcohols (such as Performacol 35O (having an average carbon chain length of 24 carbons), Performacol 425 (having an average carbon chain length of 30 carbons), or Performacol 550 (having an average carbon chain length of 40 carbons); alcohol ethoxylates (such as Performathox 420 (20% by weight ethoxylation) and Performathox 450 (50% by weight ethoxylation) all available from New Phase Technology, Piscataway, NJ may be used.

For reducing whitening in sticks liquid or solid high refractive index materials may be used such as diethylhexyl 2,6-naphthalate (from C.P. Hall Co., Chicago, IL) or phenyltrimethicone (from Dow Corning Corp., Midland, MI) as well as other suitable ingredients.

Other various optional components include those described in U.S. Patents Numbers 5,019,375 to Tanner et al; 4,937,069 to Shin; and 5,102,656. Examples of such additional ingredients include fragrances, coloring agents, opacifiers, etc. in types and amounts conventionally used for such products.

These compositions are sticks made as suspensions and thickened or gelled by the combination of polyethylene and selected wax components.

The products of the invention can be made by conventional mixing techniques. The emollients are selected, weighed out and heated with stirring to about 65 degrees C. Next the wax component is added and heating is continued to a temperature in the range of 82-85 degrees C. The polyethylene is added. The mixture is cooled to about 80 degrees C and the cyclomethicone (which has been preheated to about 70 degrees C) is added. The mixture is cooled further to 75 degrees C and the antiperspirant active is added. The temperature is increased to about 80 degrees C and held there for about 10 minutes with mixing. Fragrance, an antibacterial agent, coloring, etc. are then added if desired and thoroughly mixed. The final mixture is poured into suitable containers and then passed through a cooling tunnel which is at about 4 degrees C or placed in a refrigerator for a suitable length of time on a laboratory scale. Cooling is then completed (completion of cooling can also be done at room temperature).

The composition can be rubbed onto the skin from the top surface of the container (itself fed from a reservoir of product in the container) so as to deposit an adequate amount of the cosmetic composition on to the skin. The cosmetic composition, for example, an antiperspirant/deodorant may be applied to the skin in the axillary regions to deposit sufficient amounts of antiperspirant and/or deodorant active material to reduce body malodor and/or reduce perspiration in axillary regions of the human body.

Various forms of the invention can be exemplified by the following formulations but should not be construed as limitations on the invention:

### EXAMPLES

The following Examples are offered as illustrative of the invention and are not to be construed as limitations thereon. In the Examples and elsewhere in the description of the invention, chemical symbols and terminology have their usual and customary meanings. In the Examples as elsewhere in this application (a) values for n, m, etc. in formulas, molecular weights and degree of ethoxylation or propoxylation are averages; (b) temperatures are in degrees C unless otherwise indicated; and (c) the amounts of the components are in weight percents based on the standard described; if no other standard is described then the total weight of the composition is to be inferred. Various names of chemical components include those listed in the CTFA International Cosmetic Ingredient Dictionary (Cosmetics, Toiletry and Fragrance Association, Inc., 7th ed. 1997). Mixing techniques used to make the compositions are those conventionally used in the art including those described above.

### Example PE

A 3-liter flask reactor is equipped with a manometer and stirring apparatus and is set at atmospheric pressure with constant stirring. The reactor temperature is set at 65 degrees C by thermostat, purged with nitrogen, purged with ethylene, and then charged with 1 liter of purified dry cyclohexane, 4.6 millimoles of TiCl₄, and 2.0 millimoles of Al(C₂H₅)₃. Ethylene is then fed at the rate of 1 liter/minute into the reactor. After 15 minutes, the reaction is quenched by bubbling hydrogen gas through the reaction mixture. The low molecular weight polymers (which are oligomers) are separated by fractional distillation of the product mixture at reduced pressure (200 Torr, 26,664 Pascals).

### Example 1: General Method of Making Compositions

The emollients, for example, dimethicone (for example, DC-200, 10 centistokes and/or DC-200, 350 centistokes from Dow Corning Corp.) and C12-15 alkyl benzoate (FINSOLV TN brand product) are weighed out and placed in a 600 ml beaker. Each of the other ingredients is weighed out separately. Heating with stirring is initiated for the emollients in the 600 ml beaker until the temperature is about 65 degrees. C. The wax component is then added (for example, Japan Wax Sub 525 and/ or microcrystalline wax from Ross). Heating and stirring are continued until the temperature is in the range of 82-85 degrees C. The polyethylene (for example, PERFORMALENE-400 from New Phase Technology, Piscataway, NJ) is then added with stirring. The mixture is cooled to about 80 degrees and cyclomethicone (DC-345 from Dow Corning Corp.) which has been preheated to about 70 degrees C is then added with stirring. The mixture is further cooled to about 75 degrees C and the antiperspirant active salt (for example, Reach AZZ 902 SUF aluminum zirconium salt or Reach AZP 908 from Reheis Inc., Berkeley Heights, NJ) is added with mixing. The temperature is increased to about 80 degrees C and held there for about 10 minutes with mixing. Fragrance is added and mixing is continued for 1 minute. The mixture is poured into oval containers of the type normally used for antiperspirant/deodorant products and placed in a refrigerator at about 4 degrees C for about 15 minutes. Cooling is completed at room temperature.

In some of the examples additional ingredients such as diethylhexyl 2,6-naphthalate or Performacol 350 alcohol can be added.

## Claims

1. A high efficacy stick antiperspnant/deodorant free of added stearyl alcohol and comprising in weight percent based on the total weight of the composition:
(a) 40-50% volatile cyclomethicone;
(b) 10-25% of an antiperspirant active;
(c) 1-15% of an emollient;
(d) 1-14% of polyethylene comprising one or more members selected from the group consisting of homopolymers and copolymers of polyethylene wherein the polyethylene (i) is at least 90% linear; (ii) has a molecular weight in the range of 300-3000; (iii) has a melting point in the range of 50-129 degrees C; and (iv) has a polymer backbone of CH₃CH₂-(CH₂-CH₂)ₙ-CH₂-CH₃, where n is an average number and is selected to be in the range of 10-106; and
(e) 0.3-7% of a wax as a co-gellant with the polyethylene wherein the wax has a melting point in the range of 40-97 degrees C;
provided that the ratio of wax:polyethylene is in the range of 1:1-1:10.

2. A stick as claimed in Claim 1 wherein the emollient comprises a mixture of two or more emollients.

3. A stick as claimed in Claim 1 comprising 3-12 % emollient.

4. A stick as claimed in Claim 1 wherein the emollient comprises a non-volatile silicone.

5. A stick as claimed in Claim 4 wherein the emollient comprises a (10-350) * 10⁻⁶ m²/sec (10-350 centistoke) dimethicone.

6. A stick as claimed in Claim 1 wherein the emollient is a member of the group consisting of
(a) fats and oils represented by Formula III: 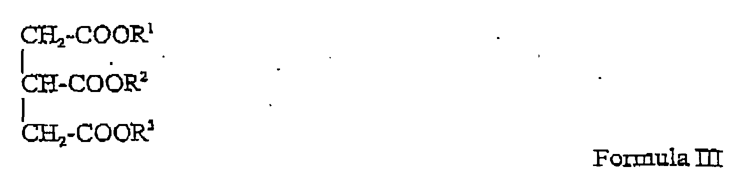 wherein each of R¹, R², and R³ may be the same or different and have a carbon chain length (saturated or unsaturated) of 7 to 30;
(b) hydrocarbons selected from the group consisting of paraffin, petrolatum, hydrogenated polyisobutene, and mineral oil;
(c) esters of general structure would be R⁴CO-OR⁵ wherein the chain length for R⁴ and R⁵ hydrocarbon groups is in the range of 7-30 and can be saturated or unsaturated, straight chained or branched;
(d) saturated and unsaturated fatty acids which have general structure R⁶COOH with the R⁶ group being a straight chain hydrocarbon with a carbon chain length between 7-10;
(e) lanolin and its derivatives selected from the group consisting of lanolin, lanolin oil, lanolin wax, lanolin alcohols, lanolin fatty acids, isopropyl lanolate, ethoxylated lanolin and acetylated lanolin alcohols;
(f) alkoxylated alcohols wherein the alcohol portion is selected from aliphatic alcohols having 2-18 carbons, and the alkylene oxide portion is selected from the group consisting of ethylene oxide, and propylene oxide having a number of alkylene oxide units from 2-53;
(g) silicones as the linear organo-substituted polysiloxanes which are polymers of silicon/oxygen with general structure:
(1) (R¹⁰)₃SiO(Si (R¹¹)₂O)ₓSi(R¹²)₃ where R¹⁰, R¹¹ and R¹² can be the same or different and are each independently selected from the group consisting of phenyl and C1-C60 alkyl; or
(2) HO(R¹⁴)₂SiO(Si (R¹⁵)₂O)ₓSi(R¹⁶)₂OH, where R¹⁴,R¹⁵ and R¹⁶ can be the same or different and are each independently selected from the group consisting of phenyl and C1-C60 alkyl; and
(h) mixtures and blends of two or more of the foregoing.

7. A stick as claimed in Claim 1 comprising 3-10% polyethylene.

8. A stick as claimed in Claim 1 wherein the polyethylene has a melting point in the range of 50-70 degrees C.

9. A stick as claimed in Claim 1 wherein the Polyethylene has a melting point in the range of 60-70 degrees C.

10. A stick as claimed in Claim 1 where the polyethylene has a melting point in the range of 70-129 degrees C.

11. A stick as claimed in Claim 1 wherein the wax has a melting point in the range of 40-80 degrees C.

12. A stick as claimed in Claim 1 wherein the wax is a microcrystalline wax having a melting point in the range of 60-97 degrees C.

13. A stick as claimed in Claim 1 additionally comprising an effective amount of an antimicrobial agent.

## Patentansprüche

1. Hochwirksamer Antitranspirant/Deodorantstift, der frei von zugesetztem Stearylalkohol ist und, bezogen auf das Gesamtgewicht der Zusammensetzung, umfasst:
(a) 40-50 Gew.-% flüchtiges Cyclomethicon;
(b) 10-25 Gew.-% Antitranspirantwirkstoff;
(c) 1-15 Gew.-% Aufweichmittel;
(d) 1-14 Gew.-% Polyethylen, das ein oder mehrere Mitglieder ausgewählt aus der Gruppe bestehend aus Homopolymeren und Copolymeren von Polyethylen umfasst, wobei das Polyethylen (i) mindestens 90 % linear ist; (ii) ein Molekulargewicht im Bereich von 300 bis 3000 hat; (iii) einen Schmelzpunkt im Bereich von 50 bis 129°C hat und (iv) ein Polymergrundgerüst aus CH₃CH₂-(CH₂-CH₂)ₙ-CH₂-CH₃, hat, wobei n eine Durchschnittszahl ist und im Bereich von 10-106 ausgewählt ist, und
(e) 0,3-7 Gew.-% Wachs als Cogeliermittel mit dem Polyethylen, wobei das Wachs einen Schmelzpunkt im Bereich von 40-97°C hat;
mit der Maßgabe, dass das Verhältnis von Wachs:Polyethylen im Bereich von 1:1 bis 1:10 liegt.

2. Stift nach Anspruch 1, wobei das Aufweichmittel eine Mischung aus zwei oder mehr Aufweichmitteln umfasst.

3. Stift, nach Anspruch 1, der 3-12 Gew.-% Aufweichmittel umfasst.

4. Stift nach Anspruch 1, wobei das Aufweichmittel nichtflüchtiges Silikon umfasst.

5. Stift nach Anspruch 4, wobei das Aufweichmittel Dimethicon mit 10-350 x 10⁻⁶ m²/s (10-350 centistoke) umfasst.

6. Stift nach Anspruch 1, wobei das Aufweichmittel ein Mitglied aus der Gruppe ist, die aus den Folgenden besteht:
(a) Fetten und Ölen mit der Formel III: 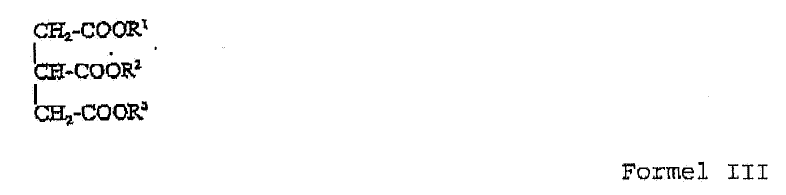 wobei jeder von R¹, R² und R³ gleich oder verschieden sein kann und eine Kohlenstoffkettenlänge (gesättigt oder ungesättigt) von 7 bis 30 hat;
(b) Kohlenwasserstoffen ausgewählt aus der Gruppe bestehend aus Paraffin, Petrolatum, hydriertem Polyisobuten und Mineralöl;
(c) Estern mit der allgemeinen Struktur R⁴CO-OR⁵, wobei die Kettenlänge für R⁴- und R⁵-Kohlenwasserstoffgruppen im Bereich von 7-30 liegt und gesättigt oder ungesättigt, geradkettig oder verzweigt sein kann;
(d) gesättigten und ungesättigten Fettsäuren, die die allgemeine Struktur R⁶COOH haben, wobei die R⁶-Gruppe ein geradkettiger Kohlenwasserstoff mit einer Kohlenstoffkettenlänge von 7 bis 10 ist;
(e) Lanolin und seinen Derivaten ausgewählt aus der Gruppe bestehend aus Lanolin, Lanolinöl, Lanolinwachs, Lanolinalkoholen, Lanolinfettsäuren, Isopropyllanolat, ethoxyliertem Lanolin und acetylierten Lanolinalkoholen;
(f) alkoxylierten Alkoholen, wobei der Alkoholanteil ausgewählt ist aus aliphatischen Alkoholen mit 2-18 Kohlenstoffatomen und der Alkylenoxidanteil ausgewählt ist aus der Gruppe bestehend aus Ethylenoxid und Propylenoxid mit einer Anzahl der Alkylenoxideinheiten von 2-53;
(g) Silikonen als lineare organosubstituierte Polysiloxane, die Polymere von Silicium/Sauerstoff mit der folgenden allgemeinen Struktur sind:
(1) (R¹⁰)₃SiO(Si(R¹¹)₂O)ₓSi(R¹²)₃, wobei R¹⁰, R¹¹ und R¹² gleich oder unterschiedlich sein können und jeweils unabhängig ausgewählt sind aus der Gruppe bestehend aus Phenyl und C₁- bis C₆₀-Allkyl; oder
(2) HO(R¹⁴)₂SiO(Si(R¹⁵)₂O)ₓSi(R¹⁶)₂OH, wobei R¹⁴, R¹⁵ und R¹⁶ gleich oder unterschiedlich sein können und jeweils unabhängig ausgewählt sind aus der Gruppe bestehend aus Phenyl und C₁- bis C₆₀-Alkyl;
(h) Mischungen und Gemische von zwei oder mehrere der Vorhergehenden.

7. Stift nach Anspruch 1, der 3-10 Gew:% Polyethylen umfasst.

8. Stift nach Anspruch 1, wobei das Polyethylen einen Schmelzpunkt im Bereich von 50-70°C hat.

9. Stift nach Anspruch 1, wobei das Polyethylen einen Schmelzpunkt im Bereich von 60-70°C hat.

10. Stift nach Anspruch 1, wobei das Polyethylen einen Schmelzpunkt im Bereich von 70-129°C hat.

11. Stift nach Anspruch 1, wobei das Wachs einen Schmelzpunkt im Bereich von 40-80°C hat.

12. Stift nach Anspruch 1, wobei das Wachs mikrokristallines Wachs mit einem Schmelzpunkt im Bereich von 60-97°C. ist.

13. Stift nach Anspruch 1, der zusätzlich eine wirksame Menge antimikrobielles Mittel umfasst.

## Revendications

1. Bâton antisudoral/déodorant à haute efficacité sans ajout d'alcool stéarylique et comprenant, en pourcentage en poids par rapport au poids total de la composition :
(a) de 40 à 50 % de cyclométhicone volatile ;
(b) de 10 à 25 % d'un agent antisudoral actif ;
(c) de 1 à 15 % d'un émollient ;
(d) de 1 à 14 % de polyéthylène comprenant un ou plusieurs composants choisis parmi le groupe consistant en les homopolymères et les copolymères de polyéthylène, dans lequel le polyéthylène (i) est linéaire à au moins 90 % ; (ii) a un poids moléculaire dans la plage de 300 à 3000 ; (iii) a un point de fusion dans la plage de 50 à 129 °C ; et (iv) a un squelette polymère de formule CH₃CH₂-(CH₂-CH₂)ₙ-CH₂-CH₃, dans laquelle n est un nombre moyen, et est choisi de façon à être dans la plage de 10 à 106 ; et
(e) de 0,3 à 7 % d'une cire en tant qu'agent cogélifiant avec le polyéthylène, dans lequel la cire a un point de fusion dans la plage de 40 à 97 °C;
à condition que le rapport cire:polyéthylène soit dans la plage de 1:1-1:10.

2. Bâton tel que revendiqué dans la revendication 1, dans lequel l'émollient comprend un mélange d'au moins deux émollients.

3. Bâton tel que revendiqué dans la revendication 1, comprenant de 3 à 12 % d'émollient.

4. Bâton tel que revendiqué dans la revendication 1, dans lequel l'émollient comprend une silicone non volatile.

5. Bâton tel que revendiqué dans la revendication 4, dans lequel l'émollient comprend une diméthicone à (10-350) x 10⁻⁶ m²/s (10-350 centistockes).

6. Bâton tel
que revendiqué dans la revendication 1, dans lequel l'émollient est un membre du groupe consistant en :
(a) les matières grasses et les huiles représentées par la formule III : 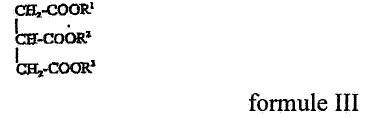 dans laquelle chacun des groupes R¹, R² et R³ peut être identique ou différent, et a une longueur de chaîne carbonée (saturée ou insaturée) de 7 à 30 ;
(b) les hydrocarbures choisis parmi le groupe consistant en une paraffine, la vaseline, le polyisobutène hydrogénée et une huile minérale ;
(c) les esters dont la structure générale serait R⁴CO-OR⁵, dans laquelle la longueur de chaîne des groupes hydrocarbonés R⁴ et R⁵ est dans la plage de 7 à 30, et peut être saturée ou insaturée, à chaîne linéaire ou ramifié
(d) les acides gras saturés et insaturés qui ont la structure générale R⁶COOH, le groupe R⁶ étant un hydrocarbure à chaîne linéaire avec une longueur de chaîne carbonée de 7 à 10 ;
(e) la lanoline et ses dérivés choisis parmi le groupe consistant en la lanoline, l'huile de lanoline, la cire de lanoline, les alcools de lanoline, les acides gras de lanoline le lanolate d'isopropyle, la lanoline éthoxylée et les alcools de lanoline acétylés ;
(f) les alcools alkoxylés dans lesquels la partie alcool est choisie parmi les alcools aliphatiques comportant de 2 à 18 atomes de carbone, et la partie oxyde d'alkylène est choisie parmi le groupe consistant en l'oxyde d'éthylène et l'oxyde de propylène, ayant un nombre de motifs oxyde d'alkylène de 2 à 53 ;
(g) les silicones telles que les polysiloxanes linéaires organo-substitués, qui sont des polymères de silicium et d'oxygène de structure générale :
(1) (R¹⁰)₃SiO(Si(R¹¹)₂O)ₓSi(R¹²)₃ dans laquelle R¹⁰, R¹¹ et R¹² peuvent être identiques ou différents, et sont choisis chacun indépendamment parmi le groupe consistant en les groupes phényle et alkyle en C₁-C₆₀ ; ou
(2) HO(R¹⁴)₂SiO(Si(R¹⁵)₂O)ₓSi(R¹⁶)₂OH, dans laquelle R¹⁴ , R¹⁵ et R¹⁶ peuvent être identiques ou différents, et sont choisis chacun indépendamment parmi le groupe consistant en les groupes phényle et alkyle en C1-C60; et
(h) les mélanges et les associations d'au moins deux des précédents.

7. Bâton tel que revendiqué dans la revendication 1, comprenant de 3 à 10 % de polyéthylène.

8. Bâton tel que revendiqué dans la revendication 1, dans lequel le polyéthylène a un point de fusion dans la plage de 50 à 70°C.

9. Bâton tel que revendiqué dans la revendication 1, dans lequel le polyéthylène a un point de fusion dans la plage de 60 à 70 °C.

10. Bâton tel que revendiqué dans la revendication 1, dans lequel le polyéthylène a un point de fusion de 70 à 129 °C.

11. Bâton tel que revendiqué dans la revendication 1, dans lequel la cire a un point de fusion de 40 à 80 °C.

12. Bâton tel que revendiqué dans la revendication 1, dans lequel la cire est une cire microcristalline ayant un point de fusion dans la plage de 60 à 97°C.

13. Bâton tel que revendiqué dans la revendication 1, comprenant, en outre, une quantité efficace d'un agent antimicrobien.
